(19) Europäisches Patentamt / European Patent Office / Office européen des brevets

(11) EP 3 943 901 A1

(12) EUROPEAN PATENT APPLICATION

(43) Date of publication:
26.01.2022 Bulletin 2022/04

(21) Application number: 20187752.9

(22) Date of filing: 24.07.2020

(51) International Patent Classification (IPC):
G01J 3/46 (2006.01)    B01F 13/00 (2006.01)
B44D 3/00 (2006.01)    A61K 8/29 (2006.01)
A61Q 1/02 (2006.01)

(52) Cooperative Patent Classification (CPC):
A61Q 1/02; A61K 2800/43; A61K 2800/59

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Cosnova GmbH
65843 Sulzbach (DE)

(72) Inventors:
• Priore, Gerardo Maria
  60311 Frankfurt am Main (DE)
• Matichard, Laure Claire Michelle
  65843 Sulzbach (Taunus) (DE)

(74) Representative: Fuchs Patentanwälte
Partnerschaft mbB
Westhafenplatz 1
60327 Frankfurt am Main (DE)

(54) **HIGH-FIDELITY COSMETIC BASIC COLOURANTS FOR USE IN HIGH-THROUGHPUT COSMETIC COLOUR MIXING MACHINES**

(57) High-fidelity cosmetic basic colourants are disclosed for the use in high-throughput cosmetic colour mixing machines. The high-fidelity cosmetic basic colourants have a MaxErrorOfX of least 2% (wt.-%). The pigments for each high-fidelity cosmetic basic colourant are mixed in a way that the minimum mass to be dispensed per base colourant X (MinOfX) and the maximum mass to be dispensed per base colourant X (MinOfX) is optimized. Optionally the high-fidelity cosmetic basic colourant comprises a coverage modifier which improves the MaxErrorOfX even further. As a result, both the basic colourants as well as the colour mixes produced by the high-fidelity basic colourants disclosed herein have a delta E (dE) of less than 2 when used in a high-throughput cosmetic colour mixing machine.

Figure 1:

## Description

Field of the Invention

High-fidelity cosmetic basic colourants are disclosed for the use in high-throughput cosmetic colour mixing machines. The high-fidelity cosmetic basic colourants have a MaxErrorOfX of least 2% (wt.-%). The pigments for each high-fidelity cosmetic basic colourant are mixed in a way that the minimum mass to be dispensed per base colourant X (MinOfX) and the maximum mass to be dispensed per base colourant X (MinOfX) is optimized. Optionally the high-fidelity cosmetic basic colourant comprises a coverage modifier which improves the MaxErrorOfX even further. As a result, both the basic colourants as well as the colour mixes produced by the high-fidelity basic colourants disclosed herein have a delta E (dE) of less than 2 when used in a high-throughput cosmetic colour mixing machine.

Background of the Invention

A trend in cosmetics is towards customization of the cosmetic product to the individual skin tone as well as the individual taste of the wearer. Colour is one parameter of such a customization.

However, it would be not economically practicable to produce a large range of very specific colours in bulk in order to match any individual skin tone, since the demand for certain colours may vary significantly.

Furthermore, in conventional colour mixing methods the bulk-container needs to be cleaned for each colour mix corresponding to a different colour blend. Thus, if a range of 1000 colour blends needs to be produced, the machine needs to be cleaned 1000 times.

Thus, in recent years, cosmetic colour mixing machines have been developed, which allow the individual mixing of cosmetic colour mixes based on cosmetic basic colourants, in order to provide a large range of individual colour mixes adjusted to the individual skin tone.

Known colour mixing machines in the prior art are for example described in WO 2014/043018, WO 2017/111589 or WO 2018/187151.

Although colour mixing machines have advantages over prior art bulk-production methods, they also give rise to a number of new problems, which were identified during the preparation of the present invention.

Detailed Description

A first problem of colour mixing machines is that they produce significantly smaller quantities of individual colour mixes as compared to conventional bulk production methods, since they prepare the colour mix within the primary package. Normally such a primary package comprises less than 100g of cosmetic composition. However, with such small volumes the problem arises that already small errors in the basic colourant (for example errors in pigment weight or colour volume distribution) can result in colour mixes which differ between different preparations (i.e. different batches of colour mixes of the same colour produced by the same machine, but at different time-points). Such deviations, however, are not accepted by the final customer for whom it is of great importance to receiving the same colour blend.

A second problem of colour mixing machines is that the machines can only distribute a basic colourant within a certain volume range. However, some basic colours need to be distributed in very low volumes in light colour blends, but in significant higher volumes in darker colour blends. Thus, on one hand, the error-margin problem, as outlined above, increases even more in cases in which only little amounts of the basic colourant are needed.

A third problem of colour mixing machines is that the "colour gamut", i.e. the colour space which can be represented by the colour mixing machine, should be as high as commercially feasible. Thus, a wide range of colour mixes needs to be provided. However, having many different receptacles comprising a wide range of different basic colourants, maybe even in different dilutions per colourant, are commercially not feasible.

A fourth problem, especially with high-throughput colour mixing machines, is that the filling process needs to be sufficiently quick in order to produce a commercially acceptable number of colour mixes per minute (e.g. 20/min or more). This high throughput, however, can only be achieved, if the numbers and volumes of the cosmetic basic colourant necessary for each colour mix do not differ too much from each other.

A fifth problem of colour mixing machines is that the volume needs for the basic colourants within a colour mix must not exceed the minimum volume that can be precisely handled by the machine or maximum volume of the primary packaging. If, for example, a basic colourant needs to be diluted so much in order to represent a certain colour blend that it exceeds the maximum volume of a primary packaging, such a basic colourant is not useful for a colour mixing machine.

Cosmetic basic colourants in the prior art do not fulfil all necessary criteria in order to be usable in high-fidelity and high-throughput colour mixing systems with satisfying results.

The present invention provides new high-fidelity basic colourants which are suitable for use in high-fidelity and high-throughput cosmetic colour mixing machines.

In a first aspect the present invention provides high-fidelity cosmetic basic colourants for use in high-throughput cosmetic colour mixing machines, wherein the cosmetic basic colourant has a MaxErrorOfX of at least 2% (wt-%), more preferred of at least 5% (wt-%), even more preferred of at least 10% (wt-%) and up to 20% (wt-%).

In one embodiment the MaxErrorOfX is 5.1% (wt-%), in another embodiment 2.01% (wt-%).

In a second aspect of the present invention the pigments for each high-fidelity cosmetic basic colourant are mixed in a way that the minimum mass to be dispensed per base colourant X (MinOfX) and the maximum mass to be dispensed per base colourant X (MaxOfX) is optimized, so that the ratio MaxOfX/MinOfX is less than 50, even more preferred less than 40, most preferred less than 35.

In a third aspect of the present invention the high-fidelity cosmetic basic colourant comprises also a coverage modifier which improves the MaxErrorOfX even further.

In a fourth aspect, as a result, the colour mixes produced by the high-fidelity basic colourants disclosed herein have a delta E (dE) of less than 2 and a weight-deviation of less than 2 wt.-% when used in a high-throughput cosmetic colour mixing machine.

The term "cosmetic basic colourant" as used herein, refers to a colourant which comprises at least one pigment and, optionally, a coverage modifier which is suitable for cosmetic use, i.e. does not pose a health risk to the final customer. At least two cosmetic basic colourants are mixed together in order to result in a "colour mix" which may be used in a cosmetic composition.

The term "cosmetic colour mixing machine" as used herein refers to any machine being able to produce a cosmetic colour mix during filling inside the primary packaging. As such the cosmetic colour mixing machine differs from conventional methods where the bulk is prepared for the final colour in comparable large volumes before it is filled into the primary packaging.

In some embodiments, however, also a manual or automated "colour mixing device" may be used to mix the basic colourants, for example as part of a kit provided with instructions to the final customer. The high-fidelity basic colourants can therefore also be used in such "colour mixing devices".

The term "colour mix" (or "cosmetic colour mix") as used herein refers to a mix of at least two high-fidelity cosmetic basic colourants mixed together in the primary packaging by the colour mixing machine or device.

Each colour within that range is called a "cosmetic colour blend" and refers to a specific colour mix which has been associated with a specific colour within the colour gamut. Such a "cosmetic colour blend" as used herein, is a colour within the convex hull described by a tetrahedron inside the space between the corners A, B, C, D. Wherein the corners A, B, C, D refer to corners of the CIELab colour space in a defined colour gamut.

|   | L (+/-1.5) | a(+/-0.2) | b (+/-0.2) |
|---|---|---|---|
| A | 79.7 | 10.7 | 18.7 |
| B | 26.0 | 10.1 | 14.0 |
| C | 38.0 | 40.8 | 38.5 |
| D | 65.9 | 15.6 | 60.5 |

The term "CIELab colour space" (also known as CIE L*a*b* or sometimes abbreviated as simply "Lab" colour space) as referred herein is a colour space defined by the International Commission on Illumination (CIE) in 1976. It expresses colour as three values: L* for the lightness from black (0) to white (100), a* from green (-) to red (+), and b* from blue (-) to yellow (+). CIELAB was designed so that the same amount of numerical change in these values corresponds to roughly the same amount of visually perceived change. The CIELAB colour space values disclosed in this document are related to the standard illuminant D55 and 2 degree measurement angles. The Lab colour values of cosmetic colour blends or colourants have to be intended as the average colour appearance of a 1ml sample stored in a black coloured receptacle of the shape of a hemisphere the volume of which is roughly 1ml (15.6mm x 15.6mm and 7.8mm deep) of such cosmetic colour blends of colourants when they are still liquid and the sample is fully opaque.

The term "dE", "delta E", "dE2000" as used herein, is the in general visual difference or distance between two colours. Common definitions make use of the Euclidean distance in a device independent colour space. The preferred definition of dE2000 makes use of a more sophisticated concept of distance in a quasi-metric space which has been adapted to solve the perceptual uniformity issue.

The nearest neighbour distance of those cosmetic blend colours, expressed in dE2000 should be less than 5, preferably less than 2, even more preferably less than 1, most preferably less than 0.5. In cases of a distance between the nearest neighbours of significant less 0.5, for example in cases of 0.25 or less, the human eye is not able anymore to differentiate between two cosmetic colour blends. In cases of a distance much higher than 2, e.g. 3 or more, the distance between different colour blends is too scarce to satisfy the customer.

Both, the basic colourants of the present invention as well as the colour mixes produced thereof in a colour mixing machine, do have a dE of less than 2, preferably less than 1.

The "MaxErrorOfX" as used herein defines the maximal deviation of weight-% of basic colourant X within a colour mix which is still visually accepted as the same colour.

Thus, a high-fidelity basic colourant suitable for colour mixing machines shows a MaxErrorOfX of at least 2% (wt-%), in one embodiment 3% (wt-%), in yet another embodiment 4% (wt-%), in yet another embodiment 5% (wt-%) or more. Basic colourants with a MaxErrorOfX of less than 1% (wt-%) are not useful for colour mixing machines since they fail to be accurately dispensed or a commercially disadvantageous number of basic colourants needs to be applied in the machine. Basic colourants with a MaxErrorOfX of more than 20% (wt-%) may also result in a need for more basic colourants in order to cover a high number of colours withing the full colour gamut.

The "MaxErrorOfX" therefore is a combination of several effects:

1. the error deviation of the machine during filling;

2. dE of the basic colourants;

3. MaxOfX/MinOfX;

4. Results of the tolerancing test.

MaxErrorOfX is the minimum of the maximum possible acceptable errors of colourant X in all colour mixes of the target gamut (i.e. **MaxErrorOfX = Min(MaxErrorOfAforX)**.

The **MaxErrorOfAforX** is the maximum possible acceptable error of basic colourant *X* within the colour mix *A* (i.e. a colourmix of the gamut G) and is calculated as such:

$$\textbf{MaxErrorOf}A\textbf{for}X = \textbf{Min}(\{Lim\text{->}\ \textbf{dE}(A,Bi)= T\ \textbf{AbsoluteValue}(fi(A,Bi))\ |\ Bi\ belongs\ to\ B\})$$

where

$fi(x,y)=1-(\textbf{ConcentrationOf}X\textbf{in}y/\textbf{ConcentrationOf}X\textbf{in}x)$, *x* and *y* colour mixes, *X* basic colourant

$dE(x,y)$ is the dE2000 between two colour mixes *x* and *y*,

*B* is a set of any colour mix *Bi* of the gamut such as that $dE(A,Bi)<T$ and

*T* is a target acceptable visual error and is measured in dE2000.

As explained before, T is preferably *2*, preferably *1.5*, preferably less than *1* and more than *0.5*.

In order for a machine (*Machine*) to be able to accurately dispense all colour mixes *A* of a given gamut *G*, being the gamut *G* a set of colour mixes, and exist a convenient *G\** set of subsets of the gamut G, *G\*= {G1, G2, G3, ..., Gn}*, such as that *G = G1* U *G2* U *G3* U...U *Gn* ("U" is the Boolean Union), then the following requirement must be met:

$$\textbf{MaxErrorOf}Machine\textbf{in}Gx < \textbf{MaxErrorOf}X = \textbf{Min}(\{\textbf{MaxErrorOf}A\textbf{for}X\ |\ for\ every\ A\ that\ belongs\ to\ Gx\})$$

for every subset *Gx* of *G* that belong to *G\**,

being **MaxErrorOf***Machine***in Gx** the maximum error of the *Machine* that dispenses colourant *X*.

MaxErrorOf*Machine*in*Gx* is measured after completing a meaningful amount of dispenses of colourant *X* (more than 10, preferably more than 100, preferably close to 1000) of the minimum possible concentration *v in* which colourant X appears in the colour mixes of gamut *Gx*.

After a meaningful amount of dispenses has been performed, the expert would have a set of volumes V={*v1, v2, v3, ..., v1000*} which are all close to the target volume *v,* which corresponds to the critical concentration of the basic colourant X for the gamut *Gx:*

$$\text{MaxErrorOf}\textit{Machine}\text{in}\textit{Gx} = \text{Max}(\text{AbsoluteValue}\textit{(v-Min(V))};\text{AbsoluteValue}\textit{(v-Max(V))}).$$

In order to create a convenient set of gamuts *G\*= {G1, G2, G3, ..., Gn}* such as that the process of calculating **MaxErrorOf***Machine***in***Gx* is convenient (fast), it is sufficient to start with *G\*={G}*. If the **MaxErrorOf***Machine***in***G* < M**axErrorOf***X* = **Min***(MaxErrorOfAforX)* is not yet satisfied for some *A\*=A1, ..., Aj* of the gamut *G*, then a new set of gamuts *G\*={G1,G2}* is created which may be suboptimal, as for instance by defining *G1=G-A\** and *G2=A\** with the use of the CIELab coordinates of every colour mix of the gamut *G*.

*If* **MaxErrorOf***Machine***in***Gx* < **MaxErrorOf***X* = **Min***(MaxErrorOfAforX)*, *for every x* being either *1 or 2,* is not yet satisfied, a new set *G\*={G1,G2,G3}* is created which may be suboptimal and so on, until *G\**, is the optimal set.

The optimal set of gamuts *G\*= {G1, G2, G3, ..., Gn}* such as above, is obtained by checking the corresponding values (in CIELab) of the colour mixes *C\*={C1, C2, C3, ..., Cn}* such as that the colour mixes in *Gx* correspond to an CIELab value in *Cx* for every *0<x<n+1* and that **Max***({dE(a,b)|* for every *a, b* that belongs to *Cx, 0<x<n+1})<T* and *T* is a target acceptable visual error and is measured in dE2000. T is preferably *2*, preferably *1.5*, preferably less than *1* and more than *0.5*.

Any suboptimal set of gamuts *G\*= {G1*, G2, G3, ..., *Gm}, m<n* is obtained by checking the corresponding values (in CIELab) of the colour mixes *C\*={C1, C2, C3, ..., Cm}* such as that the colour mixes in Gx correspond to an CIELab value in Cx for every *0<x<m+1* and that **Max***({dE(a,b)|* for every *a, b* that belongs to *Cx, 0<x<m+1})=t and t>T,* where T is the target acceptable visual error of above and is measured in dE. T is preferably *2*, preferably *1.5*, preferably less than *1* and more than *0.5.*

In a first embodiment the pigments for each basic colourant are mixed in a way that the minimum mass to be dispensed per base colourant X (MinOfX) and the maximum mass to be dispensed per base colourant X (MinOfX) is optimized. Contrary to the prior art, at least two of the inventive cosmetic basic colourants within a colour mix comprise two or more pigments in such a way that the ratio of MaxOfX/MinOfX is less than 50, in a preferred embodiment less than 40, in an even more preferred embodiment less than 35, in a most preferred embodiment 30 or less, resulting in an improved ratio MaxOfX/MinOfX and consequentially in an improved MaxErrorOfX.

A low MaxOfX/MinOfX ratio does not only improve the overall MaxErrorOfX, but also allows a high-throughput use of the basic colourants, since the volume-spread between the smallest volume needed for example for a very light colour and the largest volume needed for a very dark colour is reduced. Thus, the time needed for dispatching even large volumes like for example 30ml is kept within reasonable boundaries. In one embodiment dispatching times of less than 0.5 sec / ml are preferred, even more preferred less than 0.2 sec / ml or most preferred less than 0.15 sec / ml.

The high-throughput may also be measured in finally filled primary packages (bottles) per minute. Thus, in one embodiment a high-throughput machine is defined as being able to fill at least 30 packages/min., more preferred 40 packages/min., even more preferred 50 packages/min. or even up to 100 packages/min..

In a second embodiment the cosmetic basic colourant comprises a coverage modifier which allows to reduce the colouring power of said basic colourant, thereby increasing the MinOfX and as a result to further improve the error tolerance MaxErrorOfX. For example if a cosmetic basic colourant has a coverage of about 10% and a second cosmetic basic colourant of the same colour, but with a different coverage modifier has a coverage of 3.3%, the MaxErrorOfX is improved by replacing the first colourant with the second improved colourant from $\pm$ 1.6% to $\pm$ 5%. Furthermore, the use of the coverage-modifier instead of a simple dilution has the advantage that the volume of the cosmetic basic colourant can be kept constant as compared to conventional dilution techniques.

Furthermore, the content of coverage modifier within each cosmetic colourant may be the same or different from each other and, consequentially, the resulting colour mix may be made of cosmetic basic colourants with the same or different content of coverage modifier. Thus, further embodiments pertain to basic colourants comprising at least one coverage modifier which amount differs from the coverage modifier of at least one other basic colourant within a given colour mix. Thus, in yet another embodiment, the cosmetic colour mixes representing different cosmetic colour blends, possess different concentrations of the coverage modifiers.

It was a belief in the prior art that the coverage of the cosmetic basic colourants needs to be kept constant within all basic colourants. This is because it was believed that the higher the pigment amount in the mass the more saturated the colour looks.

It is, however, one of the inventive findings of this application that the advantage of a modified concentration of coverage modifier in the colours of the present invention outweighs the disadvantages of a less saturation. One advantage of different concentrations of the coverage modifier is that it improves the MaxErrorOfX of the basic colourant as well as of the colour mix. It was further found that darker colours can be made with less coverage and more translucency, as compared to lighter colours, but still result in cosmetic colour blends which cover sufficiently the whole colour gamut.

The modification of the colouring power does not only further improve the overall MaxErrorOfX of the basic colourant, but also further improves the applicability of high-throughput use of the basic colourant, since instead of simple dilution of the cosmetic basic colourant, the coverage modifier is used to adapt the colouring power of the colourant. Thereby keeping the volume and chemical behaviour of the basic colourant almost constant.

With such an improved MaxErrorOfX colour mixes and/or cosmetic compositions within a primary packages with an volume of between 1ml to 250ml, in some embodiments of between 5ml to 150ml, of between 10ml to 100ml, of between 25ml to 50ml can be prepared with high reliability (i.e. repeatability).

The optimization of the basic colourant including the choice of pigments and amount of coverage-modifier are outlined in the example-section.

Thus, the term "high-fidelity" as used herein refers to a cosmetic basic colourant which shows high-fidelity, i.e. a high-reproducibility in a colour mixing machine. As such, whenever such a high-fidelity cosmetic basic colourant is used in a colour mixing machine for producing a colour mix, the colour deviation between one batch of colour mix and a second batch of colour mix has a dE of less than 2, more preferred of less than 1, even more preferred of less than 0.5 and a weight-deviation of less than 5 wt.-%, more preferred of less than 2 wt.-%, even more preferred of less than 1 wt.-% and most preferred of less than 0.5 wt.-%. In one embodiment this is not only true when comparing at least one colour mix with another colour mix of the same colour, but remains within the acceptable error-margins over the whole range of colour mixes (i.e. cosmetic colour blends) within a selected colour gamut.

The term "high-throughput" is used herein for colour mixing machines which are able to produce at least 25, more preferred at least 30, more preferred at least 35, more preferred at least 40, more preferred at least 50, most preferred at least 60 or more different colour mixes and/or cosmetic compositions per minute.

The properties of the cosmetic basic colourants of the present invention possess high-fidelity, i.e. reliable repeatability, and are suitable for a reasonable production time and speed in high-throughput colour mixing machines, rendering them especially useful for such machines.

A typical colour cosmetic emulsion is composed by components selected from a list consisting of an oil phase, a water phase, emulsifiers and pigments; or any combination thereof.

The oil and water phases can be mixed together by physical means, but will start to separate again. The separation of the two phases can be delayed by the use emulsifiers (which keeps the internal phase, i.e. the phase in the lower volume, separated from the outer phase in micelles thanks to their tensioactive properties) and/or rheology additives, which, on a general level, create a mesh around the particles of one of the two phases, hence modifying drastically its rheological behaviour and the adequate manufacturing processes comprising 4 phases:

- mixing: which required the necessary hydration

- primary homogenization: which comprise the formation of the emulsion

- secondary homogenization: to reduce the particle size

- cooling

Colour pigments are solid, finely grinded materials which are dispersed (in case they are not soluble in one of the two phases) or diluted (in case they are soluble in one of the two phases) in the emulsion.

The pigments are the components of the emulsion that give the colour to the emulsion, however the other components, being not fully transparent, may influence the colour of the final emulsion as well.

There are many different pigments available in the market that differ from each other because of manufacturing process and optical properties (colour and appearance).

The "pigments" as used herein may be any colour pigment which is certified for cosmetic use. Such pigments may be of natural organic origin (e.g. animal or plant) or inorganic origin (e.g. minerals), or they may be of synthetic (i.e. chemical) origin. Examples include lapis lazuli, raw or burnt sienna, raw and burnt umber, red ochre (anhydrous $Fe_2O_3$), hydrated yellow ochre ($Fe_2O_3*H_2O$), charcoal or carbon black, blue frit (Egyptian Blue), calcium copper silicate, verdigris, indian yellow, cadmium red, prussian blue, ultramarine, cobalt blue, cerulean blue, phthalo blue, royal blue, mauveine, pigments based on azo and diazo compounds, titanium di-oxide, zinc-oxide, black oxide, red oxide, yellow oxide, pearl pigments, etc. In general any pigment listed in the colour index international which is certified for cosmetic use, can be used within this invention.

A "stable emulsion" and the terms "stable", "stability" and grammatical variations thereof, indicate a macroscopic homogeneous substance that doesn't have variation in texture or varying phases physically noticeable. In addition, microscopically the emulsion of the composition has the appearance of being homogeneous within the droplets and no dynamic changes in the microscope image are visible. Examples of compatibility that result in a stable efficacious cosmetic composition include ingredients that have compatible pH, such that the combination of the ingredients do not provide esterification, crystallization, separation, and precipitation or other incompatible effects. In addition, compatibility may include ingredients that have compatible solubility that result in a stable emulsion, wherein the ingredients do not readily break into phases. In addition, compatibility may include ingredients that retain efficacy of individual ingredients when combined with the other ingredients remain present in the formula over time periods and exposures to varying temperature.

For example, as an incompatible combination, a hydroalcoholic base is not compatible with retinal (as a second grade anti-aging agent) because the combination results in an incompatible pH and results in separation of phases. In another example, glycolic acid, lactic acid and sodium phytate (as a second grade exfoliation agent) are not compatible with the aqueous emulsion because the combination results in crystallization of the booster components. In another example, glycolic acid, lactic acid and sodium phytate (as a second grade exfoliation agent) are not compatible with tranexamic acid and urea (as a second grade whitening agent) because of a pH incompatibility.

A "good cosmetic emulsion" is considered an emulsion in which on one hand the components are stable within the emulsion and, on the other hand, is balanced in colour and composition to achieve the right organoleptic properties that the client requires. Preferably such a "good cosmetic emulsion" should also be easy to manufacture with high-throughput.

Mixing two different emulsions which formulas are not compatible, as explained above, might result in an emulsion which does not fulfill the criteria of a "good emulsion" as defined above. Such a "suboptimal" emulsion may for instance not be stable or show an inhomogeneous colour distribution.

Thus, another problem of colour mixing machines is that the basic colourants need to be composed in a way that they result in "good emulsions" over the whole range of the colour gamut within the primary packaging.

The present basic colourants fulfill this criteria, since each high-fidelity cosmetic basic colourant is compatible with all other ones. That is they can be prepared with the same proportion ratio of oil phase / water phase, the amount of dispersed pigments is similar (in some embodiments the coverage modifier serves as kind of a "replacement pigment" in the emulsion), rheology additives, etc. and are produced with the same manufacturing process.

The term "coverage" as used herein refers to the coverage of a colour cosmetic formulation as the % of basic opaque pigments to the total mass of the colour cosmetic formulation. The coverage of a formulation can be reduced without changing the formulation itself, by replacing a certain amount of opaque pigment with the same amount of coverage modifier ingredient.

Thus, a "coverage modifier" or "coverage modifier ingredient" is defined as a nearly colourless and nearly transparent pigment added to a formulation in order to reduce coverage. In this way about the same amount of solid powder is dispersed in the emulsion, but, at the same time, the amount of coverage is decreased. The advantages of using coverage modifiers are that the volume and the chemical properties can be kept nearly constant and thus, the colouring power can be adapted with neither changing the emulsifying properties nor the filling-speed of the basic colourant. In general any ingredient with the function of emphasizing the colour rendition (for example mica, talc, oils, silicones or hydro-soluble oils) may be called "coverage modifier" within this invention. Those ingredients can be replaced by the formulator instead of a pigment, without changing the equilibrium of the emulsion.

Suitable coverage modifier may be preferably selected from the mica group. The "mica group" as used herein refers to the mica group of sheet silicate (phyllosilicate) minerals including several closely related materials having nearly perfect basal cleavage. All are monoclinic, with a tendency towards pseudo-hexagonal crystals, and are similar in chemical composition. The nearly perfect cleavage, which is the most prominent characteristic of mica, is explained by the hexagonal sheet-like arrangement of its atoms.

Chemically, micas can be given the general formula $X_2Y_{4-6}Z_8O_{20}(OH, F)_4$, in which

- X is K, Na, or Ca or less commonly Ba, Rb, or Cs;

- Y is Al, Mg, or Fe or less commonly Mn, Cr, Ti, Li, etc.;

- Z is chiefly Si or Al, but also may include $Fe^{3+}$ or Ti.

Structurally, micas can be classed as dioctahedral (Y = 4) and trioctahedral (Y = 6). If the X ion is K or Na, the mica is a common mica, whereas if the X ion is Ca, the mica is classed as a brittle mica.

Thus, the coverage modifier may be selected from the list comprising: dioctahedral micas, such as muscovite, paragonite; trioctahedral micas such as common micas, biotite, lepidolite, phlogopite, zinnwaldite; brittle micas, such as clintonite; interlayer-deficient micas, such as very fine-grained micas, which typically show more variation in ion and water content (informally termed "clay micas"), they include hydro-muscovite with $H_3O^+$ along with K in the X site; Illite with a K deficiency in the X site and correspondingly more Si in the Z site; phengite with Mg or $Fe^{2+}$ substituting for Al in the Y site and a corresponding increase in Si in the Z site; and/or sericite, which is the name given to very fine, ragged grains and aggregates of white (colourless) micas. In one embodiment Mearlmica® CF (Bayer) is used.

In another embodiment also talcs may be used a coverage modifiers, as long as they are approved for cosmetic use (i.e. with less than 1% AlO$_3$ and free of asbestos and lead), such as for example ImerCare® SheerSilk, ImerCare® Velluto, ImerCare® Dolce, ImerCare® Opaline, ImerCare® 4T, ImerCare® 11T, etc.

The cosmetic composition comprising the basic colourant and/or the colour mix may comprise an array of excipients that can be mixed to achieve a custom colour, coverage and finish. Other additives may contain UV protective ingredients, vitamins, or other skincare ingredients.

**Exfoliation excipient**

In one embodiment, the excipient selected may be an ingredient that provides exfoliation to improve skin appearance. For example, in one embodiment, the actives corresponding to an excipient corresponding to an exfoliation agent include one or more of glycolic acid, lactic acid, sodium phytate, 4-(2-hydroxyethyl)-1-piperazineethanesulfonic acid (HEPES), hydroxyethyl urea, salicylic acid, citric acid, capryloyl salicylic acid, and other components that provide improvement to skin appearance, and combinations thereof.

Efficacious concentrations of the excipient selected include concentration of excipients sufficiently high to provide exfoliation. For example, efficacious concentrations of from 1 wt% to about 15 wt% of glycolic acid, lactic acid, and sodium phytate; from 0.5 wt% to about 5 wt% 4-(2-hydroxyethyl)-1-piperazineethanesulfonic acid; from 2 wt% to about 10 wt% hydroexyethyl urea; from 0.05 wt% to about 0.04 wt% salicylic acid, from 1 wt% to about 15 wt% citric acid; and from 0.01 wt% to about 0.5 wt% capryloyl salicylic acid. In one embodiment, the excipient in a cosmetic composition may include 6% 4-(2-hydroxyethyl)-1-piperazineethanesulfonic acid and 5.45% hydroxyethyl urea by weight of the skin care composition. In yet another embodiment, the excipient may include 6.7% glycolic acid, 3.3% lactic acid and 1.7% sodium phytate by weight of the skin care composition.

**Whitening excipient**

In one embodiment, the excipient selected may be an excipient that provides skin whiteness. For example, in one embodiment, the actives corresponding to an excipient corresponding to an whitening agent may include one or more of niacinamide, kojic acid, licorice extract, mulberry extract, tranexamic acid, urea, phenylethyl resorcinol, ascorbic acid, and other components that provide improvement to skin whiteness, any other suitable soluble/dispersible targeted active ingredient, and combinations thereof. Efficacious concentrations of the excipients include concentration of active excipients sufficiently high to provide whitening.

For example, efficacious concentrations of from 0.01 wt% to about 2 wt% of licorice extract; from 0.001 wt% to about 0.5 wt% mulberry extract; from 0.25 wt% to about 10 wt% niacinamide; from 0.5 wt% to about 5 wt% koiic acid; from 0.01 wt% to about 1 wt% phenylethyl resorcinol; from about 0.1 wt% to about 4 wt% tranexamic acid; and from about 1 wt% to about 15 wt% acorbic acid. In one embodiment, the excipient may include 0.1 % licorice extract; and 0.0025% mulberry extract, by weight of the skin care composition. In one embodiment, the excipient may include 3% niacinamide and 1 % kojic acid by weight of the skin care composition. In one embodiment, the excipient may include 0.5% phenylethyl resorcinol by weight of the skin care composition.

**Anti-Aging excipient**

In one embodiment, the excipient selected may be an ingredient that provides anti-aging. For example, in one embodiment, the actives corresponding to an excipient corresponding to an anti-aging agent may include one or more of C-beta-D-xylopyranoside-2-hydroxypropane (Pro-Xylane), retinal, peptides, caffeine, and other components that provide improvement to skin texture, any other suitable soluble/dispersible targeted active ingredient, and combinations thereof. Efficacious concentrations of the excipient may include concentration of active ingredients sufficiently high to provide anti-aging. For example, efficacious concentrations of from 0.5 wt% to about 10 wt% of C-beta-D-xylopyranoside-2-hydroxypropane; from 0.1 wt% to about 1.1 retinal; from 0.5 wt% to about 10 wt% ascorbic acid; from 0.1 wt% to about 1 wt% hyaluronic acid; from 0.01 wt% to about 1 wt% peptides; and from 0.1 wt% to about 1 wt% caffeine. In one embodiment, the excipient may include 3.5% C-beta-D-xylopyranoside-2-hydroxypropane, by weight of the skin care composition. In one embodiment, the excipient may include 0.1 % or 0.3% or 0.5% or 1.0% retinal, by weight of the anti-aging booster composition.

**Further excipients**

The cosmetic compositions noted above may further include additional excipients. For example, the cosmetic compositions may include humectants, such as glycol and glycerin, soothing ingredients, essential oils, Vitamin E, emollients or other ingredients for skin enhancement, solubilization or other beneficial or efficacious purpose.

In an embodiment, the composition of the disclosure may also contain excipients that are common in cosmetics, such as preserving agents, antioxidants, complexing agents, solvents, fragrances, bactericides, odor absorbers, vitamins, moisturizers, self-tanning compounds, and other active agents. The amounts of these various adjuvants are those conventionally used in the field under consideration, for example, from 0.01 % to 20% of the total weight of the composition. In one embodiment, the excipients and/or additives would be added to the cosmetic formulations to functionalize them for specific targeted treatments/needs.

**Additive base composition**

In addition to the plurality of excipients, additive base compositions are also provided.

Aqueous Alcohol Base Composition

One base composition suitable for use in the cosmetic compositions includes an aqueous alcohol base composition. The aqueous composition includes an aqueous composition, comprising, consisting essentially of or consisting of an alcohol, such as ethanol or denatured alcohol. In one embodiment, the base composition includes an aqueous alcohol base composition comprising about 35 wt% alcohol, balance water.

Aqueous Emulsion Base Composition

One base composition suitable for use in the cosmetic compositions includes an aqueous emulsion base composition. The aqueous emulsion base composition includes an aqueous composition, comprising, consisting essentially of or consisting of an emulsifier, such as polyacrylate crosspolymer-6. In one embodiment, the base composition includes an aqueous emulsion base composition comprising about 0.6 wt% polyacrylate crosspolymer-6. In another embodiment, the base composition includes an aqueous emulsion base composition comprising the following composition:

The base compositions may include components suitable for providing skin care benefits, such as moisturization, protection of skin barrier, or other skin benefit. In one embodiment, the base compositions are contained in dispensing dosing receptacles.

The concentration of the ingredients for the base compositions in the dispensing dosing receptacles is preferably at the concentrations indicated in table 1, wherein the base compositions include ranges of ingredients that permit dilution of the cosmetic compositions to their efficacious concentrations, while maintaining the benefits of the cosmetic composition.

**Table 1 - Exemplary depiction of base compositions**

| Name | Concentration (% by wt of base composition) | Concentration range (% by wt of base concentration) |
|---|---|---|
| disodium EDTA | 0.1 | 0.05 - 0.2 |
| fatty compound | 2 | 0.5 - 3 |
| polymer 1 | 0.15 | 0.1 - 1 |
| polymer 2 | 0.5 | 0.05 - 1.2 |
| polymer 3 | 0.6 | 0.5 - 2.5 |
| preservative | 0.7 | 0.1 - 1.5 |
| silicon | 2 | 0.5 - 2.5 |
| solvent 1 | 3 | 1 - 4 |
| solvent 2 | 4 | 1 - 5 |
| solvent 3 | 3 | 0.5 - 4.5 |
| solvent 4 | 0.3 | 0.05 - 1 |
| surfactant | 0.5 | 0.1 - 1 |
| water | 86 | QS |

In one embodiment the present invention also pertains to the method for producing a cosmetic composition according to the following the steps:

a. Measure the skin colour of a subject with a skin scanner device (e.g. in RGB from a CCD chip or as the spectral reflectancy map from a spectrometer);

b. Matching colour in the L*a*b colour space to the skin colour (e.g. in RGB or as a spectral reflectancy map) as measured in step a;

c. Identifying the cosmetic basic colourants resulting in a colour mix corresponding to the point in the L*a*b colour space defined in step b;

d. Mixing the above mentioned cosmetic basic colourants in a high-fidelity and high-throughput cosmetic colour mixing machine in order to receive the cosmetic colour mix matched to the skin colour as measured in step a.

e. (Optionally) adding at least one excipient to the cosmetic colour mix

f. Receiving the cosmetic composition.

**Dispenser System**

In one embodiment, a system for forming a cosmetic composition is provided. The system includes a dispensing module in selective fluid communication with a plurality of dispensing dosing receptacles mounted on a movable carousel. The dispensing module is configured to selectively dispense a plurality of the basic colourants, optionally together with additional excipients, resulting in a pharmaceutical formulation corresponding to a compatibility profile and consumer skin condition.

In another embodiment, a colour management system, including a device configured to extract one or more significant colour features corresponding to one or more skin colours captured in a plurality of digital images or by a camera sensor. In another embodiment, the skin care system includes circuitry configured to generate cosmetic formulation compatibility information based on one or more inputs associated with extracting the one more significant features. In another embodiment, the skin care system includes circuitry configured to virtually display user-specific compatible cosmetic formulation information based on at least one parameter associated with colour information and the cosmetic formulation compatibility information.

In another exemplary embodiment, a system for selecting and dispensing a cosmetic material is provided. The system includes a dispensing module configured to hold a plurality of dispensing dosing receptacles, the plurality of dispensing dosing receptacles include at least two cosmetic basic colourants and, optionally, an excipient. The dispersing arrangement being arranged and disposed to dispense at least two cosmetic basic colourants and, optionally, at least one excipient into a receiving receptacle. The system further includes a user interface configured to receive a skin profile from a user and circuitry configured to receive the skin profile received at the user interface, determine a skin colour from the skin profile, and generate a compatibility profile, determine a formulation of at least two cosmetic basic colourants and, optionally, at least one excipient, that satisfies both the skin condition and the compatibility profile, transmit the formulation to the dispensing apparatus, and control the dispensing apparatus to dispense the formulation from the plurality of dispensing dosing receptacles.

In another embodiment, a system for forming a cosmetic composition is provided. The system includes a dispensing module in selective fluid communication with a plurality of dispensing dosing receptacles. The dispensing module is configured to selectively dispense a plurality of cosmetic basic colourants with, optionally, an excipient in a controlled manner responsive to one or more inputs associated with a compatibility profile. The system further includes a skin profile module comprising skin information regarding a target skin area of a user. The system also includes a skin condition module including circuitry configured to determine a skin condition responsive to one or more inputs indicative of consumer-specific skin condition. The system further includes a target formulation including circuitry configured to determine and generate a formulation including at least two cosmetic basic colourants, and, optionally, at least one excipient, corresponding to the target skin condition determined by the skin condition software module. The system further includes a device driver module including circuitry configured to direct the dispensing module to dispense the formulation to form a stable composition having efficacious concentrations of active ingredients, such as colours and, optionally, further excipients.

In another exemplary embodiment, a method for managing colour mixing system based on a skin colour and/or condition, including generating, a user interface including one or more visual representations of a categorical skin colour, each of the visual representations corresponding to a colour defined in a colour gamut within the CIE L*a*b-colour space.

The method additionally includes generating, one or more additional scores responsive to one or more inputs indicative of a user-selected categorical skin condition. The method additionally includes generating a skin treatment regimen information including a cosmetic composition based on one or more inputs associated with the skin colour and one or more inputs indicative of a compatibility information associated with one or more cosmetic composition.

The one or more inputs indicative of a user-selected categorical skin condition may determine scores for categories of concern, such as, for example: pigmentation concern, sensitivity, complexion, wrinkles, texture, hydration, or oily/dry skin. In one particularly suitable embodiment, consultation from a skin care professional may be utilized. The skin care consultant may provide direction relating to the consumer's current regimen, skin type, skin concerns and lifestyle. To assess top skin concerns, diagnostic tools which may include, but are not limited to, a combination of a special prescription card (ranking of concerns) and reference pictures (skin atlas) are utilized. In addition, the skin information and the skin condition may be determined utilizing automated systems. For example, different devices for performing the skin diagnosis are readily understood in the art, such as the Lancome Diagnos ABS, HR Skinscope, Biotherm Bluesmart, Kiehl's Skinprofiler V.O, CA Dermanalyzer, and the Vichy Vichyconsult.

The method to achieve the detection of skin colour, in order to determine the colour of a cosmetic product adapted to the skin of a wearer of the cosmetic product, may include the following steps:

- supply data related to the colour appearance of the wearer's skin (e.g. by using a skin colour detection device as disclosed for example in application: FR1915511 and FR1915512)

- determine the colour of the cosmetic product by using a correspondence function applied to data about the colour appearance of the wearer's skin, the correspondence function associating a wearer's skin colour with a colour of the cosmetic product, the correspondence function having at least three reference associations, a reference association associating a predetermined cosmetic product colour with a predetermined wearer skin colour within a tolerance, the tolerance being equal to a distance of 6 in the CIE L*a*b* colour space and the at least three associations being chosen from the group of associations composed of:

  ◦ a first association associating the colour of the cosmetic product with coordinates in the CIE L*a*b* colour space equal to (25.9; 10.1; 14.0) with a wearer's skin colour with coordinates in the CIE L*a*b* colour space equal to (25.9; 10.1; 14.0) +/- (3.5; 3.5; 3.5),

  ◦ a second association associating the colour of the cosmetic product with coordinates in the CIE L*a*b* colour space equal to (36.9; 14.0; 22.7) with a wearer's skin colour with coordinates in the CIE L*a*b* colour space equal (36.9; 14.0; 22.7) +/- (3.5; 3.5; 3.5),

  ◦ a third association associating the colour of the cosmetic product with coordinates in the CIE L*a*b* colour space equal to (50.9; 16.2; 23.7) with a wearer's skin colour with coordinates in the CIE L*a*b* colour space equal to (50.9; 16.2; 23.7) +/- (3.5; 3.5; 3.5),

  ◦ a fourth association associating the colour of the cosmetic product with coordinates in the CIE L*a*b* colour space equal to (50.6; 22.4; 35.5) with a wearer's skin colour with coordinates in the CIE L*a*b* colour space equal to (50.6; 22.4; 35.5) +/- (3.5; 3.5; 3.5),

  ◦ a fifth association associating the colour of the cosmetic product with coordinates in the CIE L*a*b* colour space equal to (79.7; 10.7; 18.7) with a wearer's skin colour with coordinates in the CIE L*a*b* colour space equal to (79.7; 10.7; 18.7) +/- (3.5; 3.5; 3.5).

According to particular embodiments, the determination method comprises one or several of the following characteristics taken in isolation or in any technically possible combination:

- the correspondence function has at least three reference associations, each reference association associating a predetermined cosmetic product colour with each predetermined wearer's colour, within a tolerance, the at least five associations being chosen from the group of associations;

- the tolerance is equal to a the nearest neighbour distance of each cosmetic blend colour, expressed in dE2000 at less than 6, preferably less than 2, preferably less than 1, even more preferably less than 1, most preferably less than 0.5. distance in the CIE L*a*b* colour space;

- skin data are RGB-measurements made on one or several areas of the wearer's skin;

- the number of skin areas measured is greater than or equal to 3.

The use of the high-fidelity cosmetic basic colourants in a high-throughput colour mixing machine is encompassed, as well as the use of the cosmetic basic colourants and the colour mix to produce a cosmetic composition.

The cosmetic basic colourant, the colour mix and/or the cosmetic composition may be part of a kit. Such a kit may further include a receptacle and/or device in order to enable the customer to mix the basic colourant, colour mix or cosmetic composition to the customers final needs, e.g. by further adding basic colourants, colour mixes, cosmetic compositions, coverage-modifiers, excipients and/or additives, and combinations thereof, resulting in a final cosmetic composition. The kit may further comprise a device for application of the final cosmetic composition to the customer's skin. The kit may further comprise one or more items selected from the group comprising a manual or user guide, a skin scanning device, a colour scheme, a manual, a mirror, make-up removal composition, make-up removal fabrics (such as a mini-towel, a sponge, a cotton swab, a pad, etc.), detergents for cleaning the mixing device and/or applicator and skincare boosters; and any combination thereof.

Short Description of the Figures

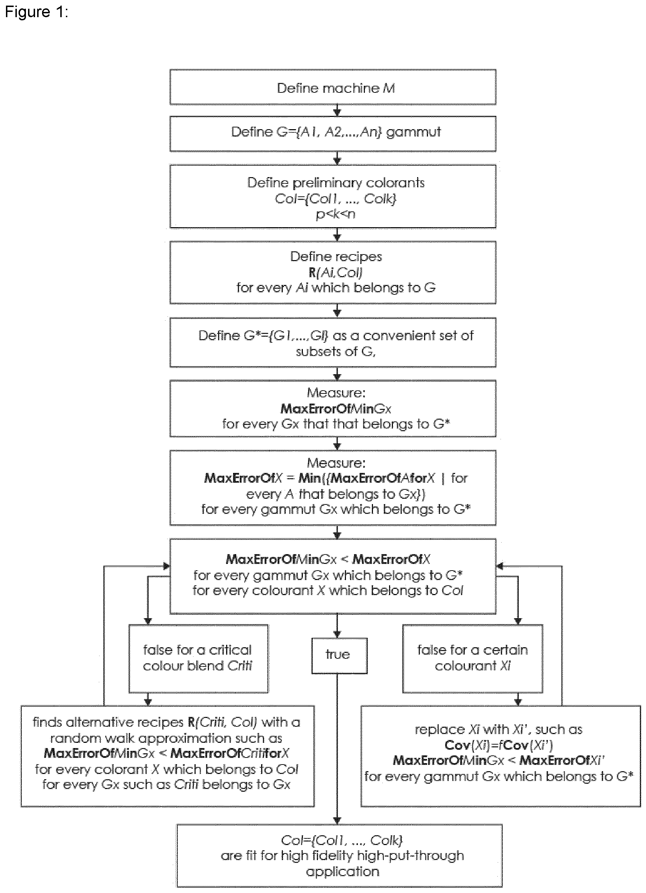

Figure 1 - Flow chart of the production and optimization of the high-fidelity cosmetic basic colourants for use in high-throughput cosmetic colour mixing machines.

**Examples**

**Example 1: Optimization of Colourants from a given colour gamut and a given machine**

In general the identification of the correct basic colourant is a multistep process.

In one embodiment the method for finding a high-fidelity cosmetic basic colourant consists of the steps:

a. Defining the error-parameters of the high throughput colour mixing machine (doser volume, distribution error, speed, number of dosers, etc.), in order to define the error-boundaries of the machine (corresponds to Example 1.1.) in the section below);

b. Defining a colour gamut G, by selecting a finite number of colour cosmetic blends (e.g. by customer evaluation)

$$G = \{G1, G2, G3, ..., Gn\}$$

each of them having average CIE Lab coordinates expressed by the colours $C=\{C1, C2, C3, ..., Cn\}$ such as $Gi$ has CIE Lab coordinates $Ci$ for $0 < i < n+1$ (corresponds to Example 1.2.) in the section below);

c. Preparing preliminary basic colourant compositions:

$$Col = \{Col1, Col2, Col3, ..., Colk\}, p < k < n$$

suitable to cover the selected colour gamut, being n the number of colour blends of the gamut G and p the number of basic pigments comprised in the cosmetic blends;

A basic pigment can be one of the commercially available pigments or a mix of commercially available pigments premixed in a given ratio and always presents in this given ratio, with the purpose of being used as a standalone pigment; (corresponds to Example 1.3.) in the section below);

d. Defining the visual error-boundaries in a tolerancing model based on the colour mixes within the colour gamut: In order to do this, the preliminary colourants Col are checked whether they can reproduce the gamut G in a way that for every colour blend A of the gamut G there is always at least a recipe of basic colourants that results in a colour mix B, the colour of which is as close as possible to the colour of colour blend A. If B exists then:

$$R (A, Col) = c1*Col1 + c2*Col2+...+ cn*Coln, c1 +...+ ck = FV$$

The linear combination of the colourants with parameters $c_1,..., c_n$, where $c_i$ is the concentration of colourant $Col_i$ is used to create the colour mix B for $0 < i < k + 1$. FV being the filling volume of the receptacle. R (A, Col) is the recipe of A with colourants Col of concentrations $c_1, ..., c_k$; (corresponds to Example 1.4.) in the section below);

e. Identify the most critical colourant pairs A/B of all basic colourants within all colour mixes of the colour gamut, based on which A/B pair violates most the error-boundaries of the colour mixing machine as defined in step a.) and/or violates most the error-boundaries of the tolerancing model:

Given the machine-error $M$ of step a. the **MaxErrorOf**$MinGx$, for every $Gx$ that belongs to $G^*$, $G^*$, being a subset of gamut $G$ which is either optimal or suboptimal as explained above (corresponds to Example 1.5.) in the section below).

f. Modify the preliminary basic colourant compositions by improving the **MaxErrorOf**$X$ in order to receive high-fidelity cosmetic basic colourants:

If **MaxErrorOf**$X$ is not verified for a certain colourant $X$ which belongs to the preliminary set of colourants $Col$, then increase **MaxErrorOf**$X$ = **Min**$(\{$**MaxErrorOf**$AforX$ | for every $A$ that belongs to $G\})$, by improving the composition of the colourant $X$.

This is done by obtaining the optimized colourant $X'$, such as **MaxErrorOf**$MinGx$ < **MaxErrorOf**$X'$ = **Min**$(\{$**MaxErrorOf**$AforX'$ | for every $A$ that belongs to $Gx\})$ for every $Gx$ that belongs to $G^*$ as defined in step e. Hence creating a new set of colourants $Col'$ such as $X'$ belongs to $Col'$ and $Col'$ contains all colourants of $Col$ but not $X$. Proceed in this way:

Being **R**$(A, Col)$, the recipe of colour blend $A$, to create $X'$ one must proceed as following:

1. Improve **MaxErrorOf**$X$ of a factor $f$, $f$= **MinOf**$M$ / **MinOf**$X$ where **MinOf**$M$ is the minimum volume $v$ which can be dispensed by the machine $M$ with an error $E$, such as $E$<**MaxErrorOf**$X$, by replacing $X$ with $X'$, a colourant with a coverage $1/f$ times the coverage of $X$; **Cov(X)=fCov(X')**

2. The following condition needs to be met:

**MaxErrorOf**$MinGx$ < **MaxErrorOf**$Coli$,

being $Coli$ any colourant of $Col$ which is not $X$.

Being $c_1', ..., c_k'$ the parameters of the recipe **R**$(A, Col')$, being $c_1, ..., c_k$ the parameters of the recipe **R**$(A, Col)$ for a given colour blend $A$, then $c_1'+...+c_k'=c_1+...+c_k=FV$. Since **MaxErrorOf**$MinGx$< **MaxErrorOf**$Coli$ depends on the concentrations of colourant $Coli$ which belongs to $Col'$ across the colour blends of the gamut $Gx$, the concentrations $c_i'$ are calculated as follow:

$c_i'=c_i*f=c$ where $c_i'$ is the concentration of $X'$ in $A$

$c_j'=(c_j*FV)/($**Sum**$(\{c_h$ | $c_h$ is the concentration of $Colh$ in $A$ and is not $c\})+c)$ where $c_j'$ is the concentration of $Colj$ in $A$, $Colj$ different from $X'$

(corresponds to Example 1.6.) in the section below).

wherein in step f.) at least one of the following optimizations is performed:

- Improve the MinOfX by adapting the pigments within the basic colourant composition, thereby keeping MaxOfX below the maximal allowable volume.

- Improve the MinOfX by adapting the concentration of the coverage modifier within the basic colourant composition, thereby keeping MaxOfX below the maximal allowable volume.

g. (optionally) If **MaxErrorOf**$X$ not verified for certain critical colour blends, the most critical colours $Crit=\{Crit1, Crit2, Crit3, ..., Critz\}$, $Crit$ are considered as a subset of $G$, such as that, if $Y$ belongs to $Crit$, then there is a colourant $X$ such as **MaxErrorOfMinGx > MaxErrorOf**$YforX$ and, if $Z$ is a colour blend of the gamut $G$ such as that **MaxErrorOf**$MinGx$ > **MaxErrorOf**$ZforX$, then $Z$ belongs to $Crit$.

Then the most critical colours are further improved by adapting the high-fidelity cosmetic basic colourants mixture based on a random walk approximation based on the basic pigments within the basic colourant.

This is possible because the preliminary colourants *Col={Col1, Col2, Col3, ..., Colk}*, where built such as that *p<k<n,* in particular *p<k,* where *p* is the number of the basic pigments. Hence for a given colour blend which belongs to *Crit,* there are more than one representation of said colour blend expressed as linear combinations of the preliminary colourants (corresponds to Example 1.7.) in the section below).

In detail:

**Example 1.1. The parameters of the colour mixing machine need to be defined.**

Depending on the dispensing technology and the commercial targets the important parameters of the colour-mixing machine need to be defined, such as dispensing volume ranges, stroke and diameter (for a volumetric pump), tube size, engine, rollers geometry (in case of a peristaltic pump) etc.

For example, the dispensing error of the minimal volume will define the error margin of the basic colours which are to be dispensed in each colour gamut.

Another important parameter is the dosing speed when dispensing large volumes. Thus, the speed threshold defined the maximum volume a basic colour may have within any of the planned colour gamuts.

Also the maximum number of available dosers belonging to the colour mixing machine need to be defined on commercial calculations and technical specifications.

The number of basic colourants should be the same or lower than the number of available dosers.

The behaviour of a certain high precision valve and its ability to reproduce a filling mass of 0.1g (=0.09ml) and 0.01g (=0.009ml) was observed in a filling test. The coefficient of variation was 6.4% (with 0.1g) and 37.7% (with 0.01g).

The coefficient of variation of the high precision valve with pure medium like water or oil may be much better than the coefficient of variation of colourant medium itself. The colourant may show adhesion to the walls of the valve chamber and air bubbles trapped in the emulsion. These are, unfortunately, properties that are important to customer satisfaction, because they define, respectively, lasting and texture of the final mix.

Thus, the coefficient of variation (i.e. the error of the machine) needs to be tested either with a "dummy colourant" or otherwise adjusted to the real situation.

**Example 1.2. A colour gamut is defined**

The colour gamut, i.e. the ideal number and distribution of colours within a given colour range is defined and may be based on commercial interest and evaluation of customer's expectations.

**Example 1.3. Preliminary basic colourants are defined**

An expert colourist submits a proposal and created a set of preliminary basic colourants. It is important that the number of basic colourants is at least one more than the number of basic pigments used. As for instance colourant-set1 comprises C1, C2, C3, C4, C5 which contains pigments P1, P2, P3, P4 in the concentrations (wt.-%) as outlined in table 2.

Table 2.

| | | Pigments | | | |
|---|---|---|---|---|---|
| | | P1 | P2 | P3 | P4 |
| Colourants | C1 | 0,28% | 7,46% | 0,03% | 0,10% |
| | C2 | 1,21% | 6,44% | 0,16% | 0,32% |
| | C3 | 1,72% | none | 6,12% | 1,89% |
| | C4 | 9,80% | none | none | none |
| | C5 | none | none | none | 9,80% |

C4 and C5 are, respectively, the pigments P1 and P4 dispersed in the emulsion of the final mixes in a roughly 10% concentration, while C1, C2, C3 are mixes of P1, P2, P3, P4.

It has been found that a minimum of 5 basic colourants is needed to avoid problems during the later tolerancing step and partly in the MinOfX.

**Example 1.4. Reproduction of the gamut with the given colourants expressed in concentration (ml) of the given colourants**

As outlined in table 3, the following colour gamut can be created from the basic colourants C1 - C5 depicted above.

Table 3.

| Original Gamut (Tot=24) | | | | | |
|---|---|---|---|---|---|
| Benchmark | C1 | C2 | C3 | C4 | C5 |
| B1 | 7.5 | 21.4 | none | none | 1.07 |
| B2 | 1.76 | none | 5.29 | 5.29 | 17.6 |
| B3 | none | none | 8.33 | 5 | 16.7 |
| B4 | none | none | 15 | 4.29 | 10.7 |
| B5 | 2.14 | none | 11.8 | 5.36 | 10.7 |
| B6 | none | none | 23.1 | 4.62 | 2.31 |
| B7 | 1.3 | none | 26.1 | 2.61 | none |
| B8 | 10.9 | 13.6 | 1.36 | 0.45 | 3.64 |
| B9 | 13.5 | 12 | 0.5 | 1.5 | 2.5 |
| B10 | 3.27 | 21.8 | 1.64 | 0.55 | 2.73 |
| B11 | 4 | 16 | 0.8 | 0.8 | 8.4 |
| B12 | none | 22.7 | 2.73 | 0.91 | 3.64 |
| B13 | 10 | 10 | 3.33 | 1.67 | 5 |

(continued)

| Original Gamut (Tot=24) | | | | | |
|---|---|---|---|---|---|
| Benchmark | C1 | C2 | C3 | C4 | C5 |
| B14 | 27.3 | 2.73 | none | none | none |
| B15 | 15 | 12.9 | 0.42 | 0.42 | 1.25 |
| B16 | 12.2 | 15.8 | 1.02 | 1.02 | none |
| B17 | none | 26.1 | 1.3 | 1.3 | 1.3 |
| B18 | 6.82 | 18.2 | 0.91 | 1.82 | 2.27 |
| B20 | 12.4 | 9.78 | 1.96 | 1.3 | 4.57 |
| B21 | 16.8 | none | 2.4 | 2.4 | 7.8 |
| B22 | 15.5 | none | 4.14 | 2.07 | 8.28 |
| B23 | 9.23 | none | 5.77 | 4.62 | 10.4 |
| B24 | 10 | none | 6 | 4 | 10 |
| | | | | | |
| MaxOfX | 27.3 | 26.1 | 26.1 | 5.36 | 17.6 |
| MinOfX | 1.3 | 2.73 | 0.42 | 0.42 | 1.07 |

## Example 1.5. Definition of the MinOfX and comparing it with the Maximum Error Of the Machine (MaxErrorOf-*Machine*in*G*) in the given Gamut

The machine error for small volumes (**MaxErrorOf***Machine***in***G*) is compared with the minimal volumes of the basic colourants needed for a given colour (**MinOf**X).

In case a given basic colourant is below the error threshold of the machine, the basic colourant needs to be adapted.

At the same time the volume of the basic colourant must not exceed the MaxOfX of the machine.

Thus, for every set of possible volume between MinOfX and MaxOfX per every colour volume (X) an assessment was done.

In conventional methods the Maximum Error Of the Machine in the given Gamut is assessed by an expert in the domain of the given filling technology by experimentation: given a target volume $v$ to be assessed, a typical experimentation involves a series of $n$ dispenses of volume $v$ with the machine, where $n$ is more than 10, preferably more than 100, preferably equal or close to 1000. Given the high number of volumes $v$ that needs to be assessed, it can be a costly and tedious process, which can break the budgetary/time constraints, so is convenient to assess a small part of the possible volumes $v$ and obtain the remaining assessment with an interpolation or, even more preferably, not assessing them at all. This method of the present invention further simplifies this process in reducing the number of volumes $v$ that needs to be assessed by the expert.

## Example 1.6. Creation of a Tolerancing Model

The error margin of the machine may be acceptable if the colour variation resulting from said error is still accepted by the customer. Thus, a tolerancing model needs to be developed which defines the maximum tolerated error rather in a certain colour composition.

Thus, it is assessed whether a certain coefficient of variation might corresponds to a colour that is too different from what the customer expects. This is done by determining **MaxErrorOf***A***for***X* for every colour blend A of the gamut for all colourants X. And once we have **MaxErrorOf***A***for***X* for every colour blend $A$ of the gamut, is possible to calculate **Min(MaxErrorOf***A***for***X***)=MaxErrorOf***X* for every colourant $X$ for every colour blend $A$ of the gamut.

To determine **MaxErrorOf** for certain $A1, A2, A3, ..., AM$, colour blends of the gamut, a consumer test is done by submitting an amount of $N$ Final Mixes being the different batches of $M$ different colours $A1, A2, A3, ..., AM$ to $C$ number of final users.

This is costly when $M$ is as high as the total number of distinct colour blends of the gamut and $N$ is high enough to be meaningful. This method of the present invention simplify this process by reducing the set $A1, A2, A3, ..., AM$ to the most critical mixes.

Combined with the machine deviations the most critical mixes A/B were identified and analysed further.

In the case of the exemplary gamut for every colourant pair A and B, A/B is always 1<A/B<40 which corresponds to A=50%; B=50% and A=98%; B=2%, and that the most common is A/B=1 (see table 4).

Table 4.

| Benchmark | A/B Pairs | | | | | | | | | |
|---|---|---|---|---|---|---|---|---|---|---|
| | C1 | C1 | C1 | C1 | C2 | C2 | C2 | C3 | C3 | C4 |
| | C2 | C3 | C4 | C5 | C3 | C4 | C5 | C4 | C5 | C5 |
| B1 | 2.857 | 1 | 1 | 7 | 2.857 | 2.857 | 20 | 1 | 7 | 7 |
| B2 | 4.25 | 3 | 3 | 10 | 1.417 | 1.417 | 2.353 | 1 | 3.333 | 3.333 |
| B3 | 1 | 1.111 | 1.5 | 2.222 | 1.111 | 1.5 | 2.222 | 1.667 | 2 | 3.333 |
| B4 | 1 | 2 | 1.75 | 1.429 | 2 | 1.75 | 1.429 | 3.5 | 1.4 | 2.5 |
| B5 | 3.5 | 5.5 | 2.5 | 5 | 1.571 | 1.4 | 1.429 | 2.2 | 1.1 | 2 |
| B6 | 1 | 3.077 | 1.625 | 3.25 | 3.077 | 1.625 | 3.25 | 5 | 10 | 2 |
| B7 | 5.75 | 20 | 2 | 5.75 | 3.478 | 2.875 | 1 | 10 | 3.478 | 2.875 |
| B8 | 1.25 | 8 | 24 | 3 | 10 | 30 | 3.75 | 3 | 2.667 | 8 |
| B9 | 1.125 | 27 | 9 | 5.4 | 24 | 8 | 4.8 | 3 | 5 | 1.667 |
| B10 | 6.667 | 2 | 6 | 1.2 | 13.33 | 40 | 8 | 3 | 1.667 | 5 |
| B11 | 4 | 5 | 5 | 2.1 | 20 | 20 | 1.905 | 1 | 10.5 | 10.5 |
| B12 | 3.03 | 2.75 | 8.25 | 2.063 | 8.333 | 25 | 6.25 | 3 | 1.333 | 4 |
| B13 | 1 | 3 | 6 | 2 | 3 | 6 | 2 | 2 | 1.5 | 3 |
| B14 | 10 | 3.636 | 3.636 | 3.636 | 2.75 | 2.75 | 2.75 | 1 | 1 | 1 |
| B15 | 1.161 | 36 | 36 | 12 | 31 | 31 | 10.33 | 1 | 3 | 3 |
| B16 | 1.292 | 12 | 12 | 1.627 | 15.5 | 15.5 | 2.102 | 1 | 7.375 | 7.375 |
| B17 | 3.478 | 5.75 | 5.75 | 5.75 | 20 | 20 | 20 | 1 | 1 | 1 |
| B18 | 2.667 | 7.5 | 3.75 | 3 | 20 | 10 | 8 | 2 | 2.5 | 1.25 |
| B20 | 1.267 | 6.333 | 9.5 | 2.714 | 5 | 7.5 | 2.143 | 1.5 | 2.333 | 3.5 |
| B21 | 2.24 | 7 | 7 | 2.154 | 3.125 | 3.125 | 1.04 | 1 | 3.25 | 3.25 |
| B22 | 2.069 | 3.75 | 7.5 | 1.875 | 1.813 | 3.625 | 1.103 | 2 | 2 | 4 |
| B23 | 1.231 | 1.6 | 2 | 1.125 | 1.3 | 1.625 | 1.385 | 1.25 | 1.8 | 2.25 |
| B24 | 1.333 | 1.667 | 2.5 | 1 | 1.25 | 1.875 | 1.333 | 1.5 | 1.667 | 2.5 |
| MIN | 1 | A(%) | 50% | B(%) | 50% | | | | | |
| MAX | 40 | A(%) | 98% | B(%) | 2% | | | | | |
| MODE | 1 | A(%) | 50% | B(%) | 50% | | | | | |

The critical mixes were the ones which were expected to be the most different from the ideal A/B ratio after the machine error was applied.

The worst case (in this case is also the MaxOfA/B) and the most common cases (in this case is also the MinOfA/B). This is calculated as (WPM-BPM)/BPM; WPM = Worst_Possible_Mix = (A+worst_error)/(B-worst_error); BPM=A/B. The higher is (WPM-BPM)/BPM for a pair of A, B, the higher the chance that mixes which contains similar ratios of A/B might be failed to be accurately reproduced by the machine.

**Table 5.**

| Colourant Pairs Concentration | A | 2% | 50% |
|---|---|---|---|
| | B | 98% | 50% |
| Absolute Colour Addition | A addition on 30ml of Ideal shade (ml) | 0,012 | 0,072 |
| Absolute Colour Subtraction | B subtraction on 30ml of Ideal shade (ml) | 0,597 | 0,072 |
| Proportion Deviation | Real(Worst Case) | 3,88% | 0,5% |

A/B mixes were created of A=15ml; B=15ml and A=29.5; B=0.5 and different deviations D were simulated with a high precision scale. Then, when the mixes were finished it was assessed at which deviation D, one starts to notice a visible difference. It was assessed that the Maximum Accepted Deviation for Colourant C1 and C3 when C1/C3=1 is between 2% and 5% which is feasible by the machine.

The same was performed for A/B=1 and A/B=40 for every A, B and with deviation D=1%, D=2%, D=5%, D=10%, D=15%, D=20%, thus assessing a Maximum Accepted Deviation every time and checking if is feasible by the machine.

### Example 1.7. Colourant Optimization.

In case of some the Maximum Accepted Deviation fail to meet the machine Deviation for a certain Dispensing Target (see *step: Tolerancing Model*) or in case the machine cannot dispense certain Dispensing Target at all, it is possible to optimize the colourants.

In this case the Maximum Acceptable Deviation of +4%/-2% was too much for the mixes A/B=40, but at A/B=24 the machine starts to work at an acceptable level:

**Table 6**.

| Colourant Pairs Concentration | A | 2% | 4% |
|---|---|---|---|
| | B | 98% | 96% |
| Absolute Colour Addition | A addition on 30ml of Ideal shade (ml) | 0.024 | 0.021 |
| Absolute Colour Subtraction | B subtraction on 30ml of Ideal shade (ml) | 1.185 | 0.516 |
| Proportion Deviation | Real(Worst Case) | 3.88% | 1.8% |

The reason of this improvement was because the machine has a deviation of +1.76%/-0.29% for the 0.8g.

To optimize a colourant the colourant was reformulated such as that MinOfX is never less than 0,8g.

First of all every colourant X was checked whether MinOfX was less than 0,8g and then the colouring power was reduced by a certain factor (K).

In general: The factor K for a colourant X means that during the process of making the colourant, 1/K of the total pigment mass of the original colourant is now a translucent pigment.

*Example*: For colourant C4, which contains, before optimization, 9.8% of pigment mass (which is 100% made of pigment P1) a K=2 means that after the optimization it will contain 4.9% of P1 and 4.9% of some translucent pigment.

However, in certain cases it may occur that one basic colour is optimized for one colour mix, but since the colours are interrelated in each colour mix this or another colour may have an unacceptable MinOfX.

For example, when trying to optimize C1 into C1* (with a Factor of K=2), this is not suitable, because there are relationships between C4, C3 and C1: bringing MinOfC3 as high as 0.8g implying that MinOfC1 is lowered to 0.5g for a certain colour mix. Thus, a further optimization of C1 is no more possible without coming back to the original point. This happens when a colour mix has a very low concentration of C1 and a very high concentration of C3 and at the same time a bottle cannot contain more than 30ml: so the expert must try to find a more convenient way to produce the critical colour mixes.

### Example 2: Optimize the critical colour mix.

This is achieved by adaptation of the colour mix using a different concentration of basic colourants based on the pigment properties distributed in each basic colourant. In this aspect it is advantageous that most of the basic colourants are composed of more than one basic pigment.

For example if a basic colourant A comprises black pigment and red pigment and basic colourant B comprises black pigment and yellow pigment, the black pigment in the colour mix can be improved by using more of basic colourant B instead of basic colourant A, if the latter show the optimization problems as explained above.

This is done by random walk approximation (highlighted with *) until an acceptable error is reached (highlighted with **), resulting in a novel improved composition for the colour mix.

| Initial Shade | B7 | | | | | |
|---|---|---|---|---|---|---|
| Colourants | | | | | | |
| | C1 | C2 | C3 | C4 | C5 | Tot |
| Mass (g) | 1.304 | none | 26.09 | 2.609 | none | 30 |
| Mass % | 4% | 0% | 87% | 9% | 0% | |
| Pigments | | | | | | |
| | P1 | P2 | P3 | P4 | | |
| Mass (g) | 0.708 | 0.097 | 1.597 | 0.494 | | 2.897 |
| Mass % | 2.4% | 0.3% | 5.3% | 1.6% | | |

| New Shade | B7* | | | | | |
|---|---|---|---|---|---|---|
| Colourants | | | | | | |
| | C1 | C2 | C3 | C4 | C5 | Tot |
| Mass (g) | none* | 1.5* | 26.09* | 2.609* | none* | 30.2 |
| Mass % | 0% | 5% | 86% | 9% | 0% | |
| Pigments | | | | | | |
| | P1 | P2 | P3 | P4 | | |
| Mass (g) | 0.722 | 0.097 | 1.599 | 0.498 | | 2.916 |
| Mass % | 2.4% | 0.3% | 5.3% | 1.6% | | |

| Error (Initial - New) | | | | | | |
|---|---|---|---|---|---|---|
| | P1 | P2 | P3 | P4 | | |
| Mass (g) | -0.01 | 7E-04 | -0 | -0 | | |
| Deviation | - 2.0%** | - 0.7%** | - 0.1%** | - 0.7%** | | |
| Weighted Deviation | -2.0% | 0.7% | -0.1% | -0.7% | | |

In this example colour mix B7* was compared by eye by a colour expert with the initial colour mix B7. In visual assessment from an expert colourist no difference could be seen between the colour mixes.

Once proven that the colour mix B7* is visually identical to initial colour mix B7 the colour mix is optimized.

**Example 3: Some examples for high-fidelity cosmetic basic colourant compositions according to the present invention.**

Basic colourant of composition C1:

| Pigment | wt-% min | wt-% max |
|---|---|---|
| White Pigment | 93.8% | 95.8% |

(continued)

| Pigment | wt-% min | wt-% max |
|---|---|---|
| Black Pigment | 0.0% | 0.0% |
| Red Pigment | 0.3% | 2.3% |
| Yellow Pigment | 2.6% | 4.6% |
| Coverage Modifier | 0.0% | 0.0% |

Basic colourant of composition C2:

| Pigment | wt-% min | wt-% max |
|---|---|---|
| White Pigment | 22.7% | 24.7% |
| Black Pigment | 1.6% | 3.6% |
| Red Pigment | 4.1% | 6.1% |
| Yellow Pigment | 13.6% | 15.6% |
| Coverage Modifier | 53.0% | 55.0% |

Basic colourant of composition C3:

| Pigment | wt-% min | wt-% max |
|---|---|---|
| White Pigment | 0.0% | 0.0% |
| Black Pigment | 20.4% | 22.4% |
| Red Pigment | 5.6% | 7.6% |
| Yellow Pigment | 5.0% | 7.0% |
| Coverage Modifier | 65.0% | 67.0% |

Basic colourant of composition C4:

| Pigment | wt-% min | wt-% max |
|---|---|---|
| White Pigment | 0.0% | 0.0% |
| Black Pigment | 0.0% | 0.0% |
| Red Pigment | 33.0% | 35.0% |
| Yellow Pigment | 0.0% | 0.0% |
| Coverage Modifier | 65.0% | 67.0% |

Basic colourant of composition C5:

| Pigment | wt-% min | wt-% max |
|---|---|---|
| White Pigment | 0.0% | 0.0% |
| Black Pigment | 0.0% | 0.0% |
| Red Pigment | 0.0% | 0.0% |
| Yellow Pigment | 33.0% | 35.0% |
| Coverage Modifier | 65.0% | 67.0% |

**Example 4: Measurement of skin colour**

Measurement of skin colour of a subject was done with a skin scanner device, the skin scanner device comprises a smartphone camera, a software operated by the said smartphone processor, a smartphone accessory, structurally and functionally associated to said smartphone, comprising colour calibration targets which ensure optimal colour consistency for the application of colour calibration of a digital picture, said picture comprises said colour calibration targets. Examples for such a device are disclosed in FR1915511 and FR1915512.

**Example 5: Matching L\*a\*b colour to the skin colour.**

This was done firstly by creating a database of at least 10, more preferably 100, more preferably close or equal to 1000 colour blends, each colour blend being identified by an CIEL\*a\*b value, obtained by placing a spectrophotometer over a sample of said colour blend.

The sample of said cosmetic colour blends have to be intended as a 1ml of said colour blend stored in a black coloured receptacle of the shape of a hemisphere the volume of which is roughly 1ml (15,6mm x1 5,6mm and 7,8mm deep) of such cosmetic colour blends of colourants when they are still liquid and the sample is fully opaque.

Then a map from the CIELab space to the colour blend space, such colour blend space is of dimension k, and every colour blend is expressed as a point R={c1,..., ck} where c1,..., ck are the concentrations of colourants Col={Col1, ..., Colk} respectively, which are necessary to a colour expert to reproduce said colour blend with said colourants.

Such map is created by the means of an interpolation; **ColourBlend***(c1, ..., ck)=Lab_value.* secondly a database of at least 100, more preferably 1000, more preferably close or equal to 10000 skintones belonging to as many different human beings, each skintone being identified by an CIEL\*a\*b value, obtained by placing a spectrophotometer over a sample of said skintone. The sample of said skintone have to be intended as a portion of that person neck of dimension 20mmx20mm, more preferably 3 portions of dimension 20mmx20mm of said person's neck should be samples and their appearances, expressed as L\*a\*b value, should be averaged.

Then a map from the CIELab space to every individual is created; **Skintone***(individual)=CIELab_value.* Thirdly each of said individual is associated with their favourite colour blends, among a set of at least 10, more preferably 100, more preferably close or equal to 1000 colour blends, each colour blend being identified by an CIEL\*a\*b value as explained above. Lastly a correspondence function which maps any given skintone (identified by an CIEL\*a\*b value as above) of an individual to any given colour blend (identified by an CIEL\*a\*b value as above) with the means of a regression; **Match***(Individual)={c1, ..., ck}* where *c1, ..., ck* are the concentrations of colourants *Col1, ..., Colk,* which are expected to create the colour blend that will please such individual.

**Example 5: Matching CIE L\*a\*b colour (colour blend) to colour mix**

As a matter of an example, in the chart below are shown 40 randomly picked cosmetic blends, in particular liquid foundations, being evenly distributed in the tridimensional colour space CIE L\*a\*b. Every colour being characterized by a colour mix comprising different quantities of five high-fidelity cosmetic basic colourants: Light, Medium, Dark, Red, Yellow (L, M, D, R, Y). The sum of the base colourants is 30g because the primary packaging in which the coloured mass must be created cannot contain more than 30g of the liquid foundation.

**Table 7: Correspondence between colour blend (depicted as CIE L\*a\*b-value) and colour mix (mixed from five high-fidelity basic colourants)**

| Colour | | | Target Mass to be dispensed per Base Colourant (g) | | | | |
|---|---|---|---|---|---|---|---|
| L | a | b | L | M | D | R | Y |
| 80 | 12 | 20 | 30.0 | 0.0 | 0.0 | 0.0 | 0.0 |
| 75 | 14 | 22 | 23.3 | 6.7 | 0.0 | 0.0 | 0.0 |
| 75 | 16 | 26 | 23.1 | 4.6 | 0.0 | 0.5 | 1.8 |
| 70 | 15 | 23 | 20.0 | 10.0 | 0.0 | 0.0 | 0.0 |
| 70 | 14 | 33 | 15.0 | 9.0 | 0.0 | 0.0 | 6.0 |
| 70 | 19 | 28 | 20.0 | 6.7 | 0.0 | 1.1 | 2.2 |
| 65 | 16 | 24 | 18.4 | 6.8 | 1.9 | 1.0 | 1.9 |
| 65 | 14 | 33 | 14.2 | 3.6 | 2.0 | 0.5 | 9.7 |
| 65 | 23 | 31 | 15.0 | 2.8 | 1.6 | 2.8 | 7.9 |
| 65 | 22 | 36 | 12.7 | 2.3 | 1.3 | 2.3 | 11.3 |

(continued)

| Colour | | | Target Mass to be dispensed per Base Colourant (g) | | | | |
|---|---|---|---|---|---|---|---|
| L | a | b | L | M | D | R | Y |
| 60 | 17 | 25 | 7.9 | 20.5 | 1.6 | 0.0 | 0.0 |
| 60 | 19 | 30 | 8.8 | 13.8 | 1.3 | 1.3 | 5.0 |
| 60 | 24 | 39 | 7.5 | 6.3 | 0.6 | 2.3 | 13.3 |
| 60 | 15 | 36 | 8.0 | 5.4 | 2.7 | 0.5 | 13.4 |
| 60 | 23 | 27 | 10.2 | 17.7 | 0.0 | 2.0 | 0.0 |
| 55 | 18 | 26 | 5.2 | 18.3 | 2.6 | 1.3 | 2.6 |
| 55 | 20 | 31 | 7.0 | 7.7 | 3.5 | 2.8 | 9.1 |
| 55 | 24 | 38 | 8.8 | 8.0 | 5.1 | 6.6 | 1.5 |
| 55 | 15 | 37 | 3.3 | 3.1 | 4.4 | 0.8 | 18.3 |
| 55 | 24 | 28 | 9.5 | 7.5 | 3.4 | 4.8 | 4.8 |
| 50 | 17 | 25 | 7.6 | 6.0 | 7.6 | 2.7 | 6.0 |
| 50 | 24 | 28 | 7.1 | 5.6 | 4.6 | 6.1 | 6.6 |
| 50 | 24 | 38 | 2.2 | 2.2 | 3.7 | 4.4 | 17.6 |
| 50 | 15 | 37 | 1.4 | 1.4 | 5.5 | 1.4 | 20.3 |
| 50 | 18 | 32 | 5.2 | 2.6 | 5.2 | 3.9 | 13.0 |
| 45 | 17 | 25 | 2.0 | 14.0 | 8.0 | 2.0 | 4.0 |
| 45 | 19 | 30 | 0.0 | 13.6 | 5.5 | 2.7 | 8.2 |
| 45 | 23 | 37 | 0.0 | 2.6 | 6.1 | 4.9 | 16.4 |
| 45 | 15 | 36 | 0.4 | 0.4 | 7.2 | 2.0 | 20.0 |
| 45 | 23 | 27 | 1.9 | 13.1 | 5.6 | 5.6 | 3.8 |
| 40 | 15 | 25 | 0.0 | 6.4 | 12.9 | 2.1 | 8.6 |
| 40 | 14 | 33 | 0.0 | 0.0 | 10.6 | 2.6 | 16.8 |
| 40 | 23 | 31 | 0.0 | 0.0 | 10.2 | 6.8 | 13.0 |
| 40 | 22 | 36 | 0.0 | 0.0 | 9.2 | 5.8 | 15.0 |
| 35 | 14 | 22 | 0.0 | 6.4 | 17.1 | 2.1 | 4.3 |
| 35 | 13 | 27 | 0.0 | 2.5 | 15.8 | 2.5 | 9.2 |
| 35 | 20 | 29 | 0.0 | 2.1 | 13.6 | 6.4 | 7.9 |
| 30 | 14 | 22 | 0.0 | 0.0 | 21.4 | 2.1 | 6.4 |
| 30 | 10 | 20 | 0.0 | 0.0 | 25.0 | 0.0 | 5.0 |
| 25 | 11 | 15 | 0.0 | 0.0 | 30.0 | 0.0 | 0.0 |

**Example 6: Mixing of colourants in the primary packaging.**

The mixing of the colourants take place preferentially within the primary packaging by the means of a plate which comprises several recesses, wherein those recesses are meant to be structurally associated with said primary packagings in such a way that they are jointly liable to said plate.

Said plate moves along one or more axis. Said movement of the plate causes the mixing of the colourants which are inside the primary packaging.

Preferentially the primary packaging comprises additionally at least one heavy element such as a metal ball or a metal ring which is able to freely move inside it within the mechanical constraints of the inner cavity of the primary packaging itself.

**Example 7: Kit for Customers.**

Individuals may be allowed to create a new colour blend instead of choosing among the finite set of at least 10, more preferably at least 100, most preferably close or equal to 1000 colour blends. Said creation of a new colour blend is performed with a kit as described before.

**Claims**

1. A high-fidelity cosmetic basic colourant for use in high-throughput cosmetic colour mixing machines, wherein the cosmetic basic colourant has a MaxErrorOfX of at least 2%.

2. The high-fidelity cosmetic basic colourant according to claim 1, wherein the difference of the minimum mass to be dispersed per cosmetic basic colourant (MinOfX) in a first colour mix and the maximum mass to be dispersed per cosmetic basic colourant (MaxOfX) in a second colour mix has a MaxOfX/MinOfX-ratio of less than 50.

3. The high-fidelity cosmetic basic colourant according to claim 1 or 2, wherein the cosmetic basic colourant further comprises a coverage modifier.

4. The high-fidelity cosmetic basic colourant according to claim 3, wherein the coverage modifier is selected from the mica group.

5. The high-fidelity cosmetic basic colourant according to any of the previous claims, wherein the cosmetic basic colourant is selected from a list comprising:

a basic colourant of composition C1:

| Pigment | wt-% min | wt-% max |
| --- | --- | --- |
| White Pigment | 93.8% | 95.8% |
| Black Pigment | 0.0% | 0.0% |
| Red Pigment | 0.3% | 2.3% |
| Yellow Pigment | 2.6% | 4.6% |
| Coverage Modifier | 0.0% | 0.0% |

a basic colourant of composition C2:

| Pigment | wt-% min | wt-% max |
| --- | --- | --- |
| White Pigment | 22.7% | 24.7% |
| Black Pigment | 1.6% | 3.6% |
| Red Pigment | 4.1% | 6.1% |
| Yellow Pigment | 13.6% | 15.6% |
| Coverage Modifier | 53.0% | 55.0% |

a basic colourant of composition C3:

| Pigment | wt-% min | wt-% max |
| --- | --- | --- |
| White Pigment | 0.0% | 0.0% |
| Black Pigment | 20.4% | 22.4% |
| Red Pigment | 5.6% | 7.6% |
| Yellow Pigment | 5.0% | 7.0% |
| Coverage Modifier | 65.0% | 67.0% |

a basic colourant of composition C4:

| Pigment | wt-% min | wt-% max |
|---|---|---|
| White Pigment | 0.0% | 0.0% |
| Black Pigment | 0.0% | 0.0% |
| Red Pigment | 33.0% | 35.0% |
| Yellow Pigment | 0.0% | 0.0% |
| Coverage Modifier | 65.0% | 67.0% |

and a basic colourant of composition C5:

| Pigment | wt-% min | wt-% max |
|---|---|---|
| White Pigment | 0.0% | 0.0% |
| Black Pigment | 0.0% | 0.0% |
| Red Pigment | 0.0% | 0.0% |
| Yellow Pigment | 33.0% | 35.0% |
| Coverage Modifier | 65.0% | 67:0% |

; and any combination thereof.

6. A colour mix comprising at least two of the high-fidelity cosmetic basic colourants of according to any one of claims 1 to 5.

7. The colour mix according to claim 6, wherein the coverage value is different between at least two of the high-fidelity cosmetic basic colourants.

8. The colour mix according to claim 7, wherein the difference between the concentration of coverage modifier ingredients in the most covering of the cosmetic basic colourants (CovMax) and the concentration of coverage modifier ingredients in the least covering of the cosmetic basic colourants (CovMin) has a ratio of CovMax/CovMin of less than 2.5.

9. Cosmetic composition comprising the high-fidelity cosmetic basic colourants according to any one of claims 1 to 5, or the colour mix according to any one of claims 6 to 8.

10. Cosmetic composition according to claim 9, further comprising at least one cosmetic acceptable excipient and/or additive selected from the list of an emulsifier, an oil, an ointment, an anti-ageing agent, an exfoliation agent and/or a whitening agent.

11. Method for producing a cosmetic composition according to any one of claims 9 or 10 following the steps of:

a. Measure the skin colour of a subject with a skin scanner device;
b. Matching a point in the CIE L*a*b colour space to the skin colour as measured in step a;
c. Identifying the cosmetic basic colourants resulting in a colour mix corresponding to the point in the CIE L*a*b colour space defined in step b;
d. Mixing the high-fidelity cosmetic basic colourants according to any one of claims 1 to 5 in a high-fidelity and high-throughput colour mixing machine in order to receive the colour mix matched to the skin colour as measured in step a.
e. (Optionally) adding at least one excipient and/or additive to the colour mix
f. Receiving the cosmetic composition.

12. Use of the high-fidelity cosmetic basic colourants according to any one of claims 1 to 5 in a high-throughput cosmetic colour mixing machine.

**13.** A high-fidelity and high-throughput cosmetic colour mixing machine comprising the high-fidelity cosmetic basic colourants according to any one of claims 1 to 5, the colour mix according to any one of claims 6 to 8, or the cosmetic composition according to any one of claims 9 or 10, and any combination thereof.

**14.** A kit of parts comprising the cosmetic basic colourants according to any one of claims 1 to 5, the colour mix according to any one of claims 6 to 8, or the cosmetic composition according to any one of claims 9 or 10, and any combination thereof.

**15.** The kit according to claim 14, further comprising a device selected from a skin scanner, a mixing device, a syringe, an applicator, a colour scheme, a manual, a mirror, make-up removal, syringe detergent, skincare boosters, and any combination thereof.

Figure 1:

```
┌─────────────────────────────────────┐
│           Define machine M            │
└─────────────────────────────────────┘
                    │
                    ▼
┌─────────────────────────────────────┐
│      Define G={A1, A2,...,An} gammut  │
└─────────────────────────────────────┘
                    │
                    ▼
┌─────────────────────────────────────┐
│       Define preliminary colorants    │
│          Col={Col1, ..., Colk}        │
│                p<k<n                   │
└─────────────────────────────────────┘
                    │
                    ▼
┌─────────────────────────────────────┐
│            Define recipes             │
│              R(Ai,Col)                │
│      for every Ai which belongs to G  │
└─────────────────────────────────────┘
                    │
                    ▼
┌─────────────────────────────────────┐
│  Define G*={G1,...,Gl} as a convenient set of │
│              subsets of G,            │
└─────────────────────────────────────┘
                    │
                    ▼
┌─────────────────────────────────────┐
│               Measure:                │
│           MaxErrorOfMinGx             │
│    for every Gx that that belongs to G* │
└─────────────────────────────────────┘
                    │
                    ▼
┌─────────────────────────────────────┐
│               Measure:                │
│  MaxErrorOfX = Min({MaxErrorOfAforX | for │
│      every A that belongs to Gx})     │
│  for every gammut Gx which belongs to G* │
└─────────────────────────────────────┘
```

MaxErrorOfMinGx < MaxErrorOfX
for every gammut Gx which belongs to G*
for every colourant X which belongs to Col

false for a critical colour blend Criti

true

false for a certain colourant Xi

finds alternative recipes R(Criti, Col) with a random walk approximation such as MaxErrorOfMinGx < MaxErrorOfCritiforX for every colorant X which belongs to Col for every Gx such as Criti belongs to Gx

replace Xi with Xi', such as
Cov(Xi)=fCov(Xi')
MaxErrorOfMinGx < MaxErrorOfXi'
for every gammut Gx which belongs to G*

Col={Col1, ..., Colk}
are fit for high fidelity high-put-through application

**EUROPEAN SEARCH REPORT**

Application Number

EP 20 18 7752

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/228892 A1 (NICHOL JAMIE GORDON [US] ET AL) 10 August 2017 (2017-08-10) * claims 1,8 * | 1-3,6-15 | INV.<br>G01J3/46<br>B01F13/00<br>B44D3/00 |
| X | WO 2006/092604 A2 (CARROLL CHARLES CONRAD [GB]; COWARD TREVOR [GB]) 8 September 2006 (2006-09-08) * claims 1,6 * | 1-3,6-15 | A61K8/29<br>A61Q1/02 |
| X | US 5 622 692 A (RIGG RICHARD T [US] ET AL) 22 April 1997 (1997-04-22) * claim 1 * | 1-3,6-15 | |
| X | US 6 284 228 B1 (MARKOWITZ DAN [US] ET AL) 4 September 2001 (2001-09-04) * column 2, lines 1-6; claim 1 * | 1-15 | |
| X | Timica Terra ET AL: "Safety Data Sheet", , 19 December 2013 (2013-12-19), XP055764290, Retrieved from the Internet: URL:http://dir.cosmeticsandtoiletries.com/ file/4521/Timica %20Terra%20Black%20MN4498%20MSDS.pdf [retrieved on 2021-01-12] * paragraph ["composition"] * | 4 | |

TECHNICAL FIELDS SEARCHED (IPC)

G01J
B01F
B44F
B44D
A61K
A61Q

The present search report has been drawn up for all claims

| Place of search | Date of completion of the search | Examiner |
|---|---|---|
| Munich | 13 January 2021 | Verrucci, Marinella |

EPO FORM 1503 03.82 (P04C01)

## ANNEX TO THE EUROPEAN SEARCH REPORT
## ON EUROPEAN PATENT APPLICATION NO.

EP 20 18 7752

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2021

| Patent document cited in search report | | Publication date | Patent family member(s) | | Publication date |
|---|---|---|---|---|---|
| US 2017228892 | A1 | 10-08-2017 | AU | 2017217455 A1 | 23-08-2018 |
| | | | CA | 3013931 A1 | 17-08-2017 |
| | | | CN | 108885134 A | 23-11-2018 |
| | | | EP | 3414538 A1 | 19-12-2018 |
| | | | JP | 6574074 B2 | 11-09-2019 |
| | | | JP | 2019515759 A | 13-06-2019 |
| | | | KR | 20180109080 A | 05-10-2018 |
| | | | US | 2017228892 A1 | 10-08-2017 |
| | | | US | 2018033161 A1 | 01-02-2018 |
| | | | US | 2019080480 A1 | 14-03-2019 |
| | | | WO | 2017139319 A1 | 17-08-2017 |
| WO 2006092604 | A2 | 08-09-2006 | EP | 1856493 A2 | 21-11-2007 |
| | | | GB | 2424477 A | 27-09-2006 |
| | | | US | 2009213379 A1 | 27-08-2009 |
| | | | WO | 2006092604 A2 | 08-09-2006 |
| US 5622692 | A | 22-04-1997 | AU | 697347 B2 | 01-10-1998 |
| | | | CA | 2168558 A1 | 02-03-1995 |
| | | | DE | 69411924 T2 | 17-12-1998 |
| | | | EP | 0717657 A1 | 26-06-1996 |
| | | | ES | 2120065 T3 | 16-10-1998 |
| | | | JP | H09502022 A | 25-02-1997 |
| | | | NZ | 271658 A | 24-11-1997 |
| | | | US | 5622692 A | 22-04-1997 |
| | | | WO | 9505892 A1 | 02-03-1995 |
| | | | ZA | 946410 B | 23-02-1996 |
| US 6284228 | B1 | 04-09-2001 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014043018 A
- WO 2017111589 A
- WO 2018187151 A

- FR 1915511
- FR 1915512